# EUROPÄISCHE PATENTSCHRIFT

(11) **EP 0 387 674 B1**
(45) Veröffentlichungstag und Bekanntmachung des Hinweises auf die Patenterteilung: **09.06.1993**
(21) Anmeldenummer: 90104314.1
(22) Anmeldetag: 07.03.1990
(51) Int. Cl.: C07C 69/14, C07C 67/08, C07C 67/54

(54) **Verfahren zur kontinuierlichen Herstellung eines wasserfreien Methylacetat-Methanol-Gemisches**
Process for the continuous preparation of an anhydrous mixture of methyl acetate and methanol
Procédé de préparation continue d'un mélange anhydre d'acétate de méthyle et de méthanol

(30) Priorität: 16.03.1989 DE 3908555
(43) Veröffentlichungstag der Anmeldung: 19.09.1990
(73) Patentinhaber: HOECHST AKTIENGESELLSCHAFT, 65926 Frankfurt am Main (DE)
(72) Erfinder: Erpenbach, Heinz, Dr., D-5000 Köln (DE); Günther, Klaus, Dr., D-6239 Eppstein/Taunus (DE); Kohl, Georg, D-5030 Hürth (DE)

(56) Entgegenhaltungen:
- US-A- 4 435 595

## Beschreibung

Die vorliegende Erfindung betrifft ein Verfahren zur kontinuierlichen Herstellung eines wasserfreien Methylacetat-Methanol-Gemisches aus Essigsäure und Methanol in Gegenwart eines sauren Veresterungskatalysators.

Die Veresterung von wasserfreier Essigsäure - auch Eisessig genannt - mit Methanol in Gegenwart von sauren Veresterungskatalysatoren, wie beispielsweise Schwefel- und Phosphorsäure oder sogenannten Lewis-Säuren, zu Methylacetat gehört seit langem zum Stand der Technik (siehe "Ullmanns Encyklopädie der technischen Chemie", 3. Auflage, 1955, 6. Band, Seiten 796 bis 797). Um das Veresterungsgleichgewicht auf die Seite des Esters zu ziehen, wird das gebildete Wasser durch Destillation aus der Reaktionsmischung ausgeschleust.

In der EP-0 060 717 A1 wird ein Verfahren zur Herstellung von Methylacetat durch Veresterung von Methanol mit Essigsäure beschrieben, bei welchem mittels eines Schleppmittels Methylacetat und Wasser aus der Reaktionsmischung als Azeotrop destilliert wird. Dieses Azeotrop kann durch eine weitere Destillation gereinigt werden. Hierbei wird ein Methylacetat gewonnen, welches 1,5 Gew% Methanol, 5,2 Gew% Wasser, 0,002 Gew% Essigsäure und 0,05 Gew% n-Butylacetat (Schleppmittel) enthält. Ohne Schleppmittel wird ein Destillat erhalten, welches neben Methylacetat noch 27,9 Gew% Methanol, 13,8 Gew% Wasser und 0,8 Gew% Essigsäure aufweist.

Das gemäß der EP-0 060 717 A1 hergestellte Methylacetat kann nicht in einer Carbonylierungsreaktion zur alleinigen Herstellung von Essigsäureanhydrid eingesetzt werden, da entsprechend dem Wassergehalt in der Carbonylierungsreaktion stöchiometrische Essigsäuremengen gebildet werden. Die Carbonylierungsreaktion zur Herstellung von Essigsäureanhydrid ist in der US-A-4 046 807 vorbeschrieben.

Es bestand daher die Aufgabe, ein Verfahren zur kontinuierlichen Herstellung eines wasserfreien Methylacetat-Methanol-Gemisches anzugeben, das mit hoher Ausbeute, bezogen auf Essigsäure, kurzer Verweilzeit in der Veresterungszone und ohne Schleppmittel betrieben werden kann und dessen Abwasser in einer biologischen Reinigung problemlos gereinigt werden kann.

Die Aufgabe konnte überraschend dadurch gelöst werden, daß man die Veresterung in einer Destillationskolonne mit n = 20 bis 50 Destillationsböden durchführt, wobei man
a) die Essigsäure zusammen mit dem Katalysator auf einen Destillationsboden, dessen Abstandszahl 0,5 n bis 0,8 n, bezogen auf die Destillationsblase, beträgt, einspeist,
b) 50 bis 80 Gew% des gesamten Methanols in die Destillationsblase und das restliche Methanol auf einen Destillationsboden, der 2 bis 10 Böden oberhalb des Essigsäureeinspeisungsbodens liegt, einspeist,
c) Methanol und Essigsäure in einem Molverhältnis von (1,5 bis 3) : 1 in die Destillationskolonne einspeist,
d) ein Rücklaufverhältnis von (0,75 bis 1,5) : 1 einstellt,
e) das wasserfreie Methylacetat-Methanol-Gemisch am Kopf der Kolonne entnimmt und
f) aus der Destillationsblase einen Wasser-Methanol-Essigsäure-Sumpf mit dem Katalysator abzieht.

Das erfindungsgemäße Verfahren kann weiter noch wahlweise dadurch ausgestaltet sein, daß man
1) ein Methanol-Essigsäure-Gemisch mit einem Molverhältnis von (1,6 bis 2,0) : 1 einspeist,
2) ein Rücklaufverhältnis von (0,9 bis 1,2) : 1 einstellt,
3) die Veresterung in einer Destillationskolonne mit 30 bis 40 Siebböden vornimmt und die gesamte Essigsäure auf dem 20. bis 25. Siebboden zugibt, 60 bis 70 Gew% des insgesamt eingespeisten Methanols gasförmig in die Destillationsblase und das restliche Methanol 4 bis 6 Siebböden oberhalb der Essigsäureeinspeisung einführt,
4) als Veresterungskatalysator konzentrierte Schwefelsäure einsetzt,
5) die Destillation drucklos betreibt,
6) die Essigsäure zusammen mit dem Katalysator auf eine Temperatur von 40 bis 60°C erwärmt,
7) aus dem Wasser-Methanol-Sumpf der Destillationsblase in einer zweiten Destillation das Methanol rückgewinnt,
8) die zweite Destillation mit einem Rücklaufverhältnis von (5 bis 50) : 1, insbesondere (10 bis 15) : 1, betreibt,
9) das rückgewonnene Methanol in die Veresterungsstufe rückführt.

Grundsätzlich ist es auch möglich, die erfindungsgemäße Veresterung in Destillationskolonnen mit mehr als 50 Destillationsböden durchzuführen. Wegen der erhöhten Investitionskosten ist aber die Verwendung von mehr als 50 Destillationsböden unwirtschaftlich, da lediglich ein geringfügig erhöhter Methylacetatanteil im Methylacetat-Methanol-Gemisch resultiert. Bei Destillationskolonnen mit weniger als 20 Destillationsböden ist ein Durchschlagen von Wasser in das Kopfprodukt möglich.

Mit dem erfindungsgemäßen Verfahren ist es nunmehr möglich, ein wasser- und säurefreies (unterhalb der üblichen Erfassungsgrenze von 0,015 Gew% Wasser und 0,005 Gew% Essigsäure) Methylacetat-Methanol-Gemisch herzustellen, welches nach der gaschromatographischen Analyse lediglich einen Gesamtgehalt an Fremdkomponenten von etwa 100 ppm hat, wobei die Fremdkomponenten im wesentlichen aus Formaldehyddimethylacetat, Ethylacetat und Methylformiat bestehen. Das wasserfreie Methylacetat-Methanol-Gemisch enthält bei der Veresterung in einer Destillationskolonne mit 35 Glockenböden 20 bis 24 Gew% Methanol und entspricht damit fast der Zusammensetzung des in der Literatur angegebenen Wertes für das Azeotrop mit 18,7 Gew% Methanol.

Der Sumpf der Destillationsblase enthält das im stöchiometrischen Überschuß eingesetzte Methanol abzüglich des als Kopfprodukt ausgeschleusten Methanols, sowie 0,5 bis 3,0 Gew% Essigsäure und die als Katalysator eingesetzte Schwefelsäure.

In einer Füllkörperkolonne können die organischen Bestandteile von dem schwefelsauren Wasser abdestilliert werden. Dieses Gemisch kann vorteilhaft in die erste Destillationskolonne als Reaktant rückgeführt werden.

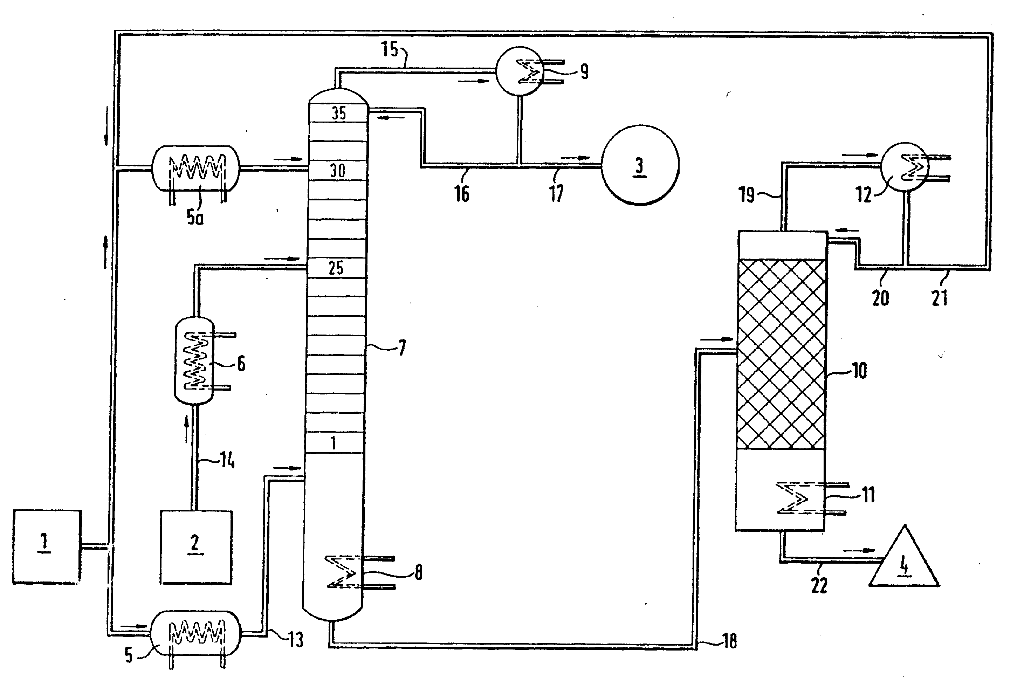

Die erfindungsgemäße Arbeitsweise wird anhand des Beispiels und der Zeichnung näher erläutert.

### Beispiel

Als Destillationskolonne (7) wurde eine Siebbodenkolonne mit einem Durchmesser von 2,5 m eingesetzt. Die Kolonne hatte 35 Siebböden. Der Bodenabstand betrug 500 mm, die Wehrhöhe auf jedem Boden 120 mm. Als Strippkolonne (10) wurde eine Füllkörperkolonne mit einem lichten Durchmesser von 750 mm und einer Höhe von 22 m verwendet.
7 730 kg/h Essigsäure und 80 kg/h 98%ige Schwefelsäure wurden aus dem Essigsäurevorrat (2) in dem Vorwärmer (6) auf 55°C erwärmt und über die Leitung (14) auf den 25. Siebboden, bezogen auf die Destillationsblase (8) der Destillationskolonne (7), eingespeist. Aus dem Methanolvorrat (1) und dem Kondensator (12) wurden über den Vorwärmer (5a) 2 560 kg/h Methanol auf den 30. Siebboden mit einer Temperatur von 50°C eingespeist. Weitere 4 500 kg/h Methanol wurden im Verdampfer (5) verdampft und gasförmig durch Leitung (13) in die Destillationsblase (8) dosiert. Das molare Verhältnis Methanol zu Essigsäure betrug somit 1,71. Die Blasentemperatur wurde auf 92,5°C gehalten. Das Kopfprodukt wurde durch die Brüdenleitung (15) dem Kondensator (9) zugeführt. Über die Rücklaufleitung (16) wurde ein Rücklaufverhältnis von 1,1 : 1 eingestellt. Die mittlere Verweilzeit für die Produkte in der Kolonne belief sich auf 0,75 Stunden. Die Kopftemperatur der Destillationskolonne (7) betrug 52°C. Über die Leitung (17) wurden 12 200 kg/h wasserfreies Methylacetat-Methanol-Gemisch (77,9/22,1 Gew%) in den Vorrat (3) abgezogen. Das entspricht einer Methylacetat-Ausbeute von 99,6 %, bezogen auf den Essigsäure-Einsatz.
Aus der Destillationsblase (8) wurden 2 670 kg/h Sumpf durch die Ablaßleitung (18) in die Mitte der Strippkolonne (10) eindosiert. Der Sumpf bestand aus 9,0 Gew% Methanol, 0,4 Gew% Methylacetat, 1,1 Gew% Essigsäure, 3,0 Gew% Schwefelsäure und Rest Wasser. Das Kopfprodukt wurde über die Brüdenleitung (19) dem Kondensator (12) zugeführt und darin kondensiert. In der Strippkolonne (10) wurde über die Rücklaufleitung (20) ein Rückflußverhältnis von 10 : 1 eingestellt. Die Kopftemperatur der Strippkolonne (10) betrug 65°C und die Blasentemperatur 100°C. Über die Leitung (21) wurden 250 kg/h Methanol in die Destillationskolonne (7) rückgeführt. Das rückgeführte Methanol enthielt 4,0 Gew% Methylacetat.
Aus der Blase (11) wurden über die Ablaßleitung (22) 2 420 kg/h Abwasser der biologischen Abwasserreinigung (4) zugeführt. Das Abwasser enthielt 1,24 Gew% Essigsäure und 3,3 Gew% Schwefelsäure.

## Patentansprüche

1. Verfahren zur kontinuierlichen Herstellung eines wasserfreien Methylacetat-Methanol-Gemisches durch Veresterung von Essigsäure und Methanol in Gegenwart eines sauren Veresterungskatalysators, dadurch gekennzeichnet, daß man die Veresterung in einer Destillationskolonne mit n = 20 bis 50 Destillationsböden durchführt und wobei man
a) die Essigsäure zusammen mit dem Katalysator auf einen Destillationsboden, dessen Abstandszahl 0,5 n bis 0,8 n, bezogen auf die Destillationsblase, beträgt, einspeist,
b) 50 bis 80 Gew% des gesamten Methanols in die Destillationsblase und das restliche Methanol auf einen Destillationsboden, der 2 bis 10 Böden oberhalb des Essigsäureeinspeisungsbodens liegt, einspeist,
c) Methanol und Essigsäure in einem Molverhältnis von (1,5 bis 3) : 1 in die Destillationskolonne einspeist,
d) ein Rücklaufverhältnis von (0,75 bis 1,5) : 1 einstellt,
e) das wasserfreie Methylacetat-Methanol-Gemisch am Kopf der Kolonne entnimmt und
f) aus der Destillationsblase einen Wasser-Methanol-Essigsäure-Sumpf mit dem Katalysator abzieht.

2. Verfahren nach Anspruch 1, dadurch gekennzeichnet, daß man ein Methanol-Essigsäure-Gemisch mit einem Molverhältnis von (1,6 bis 2,0) : 1 einspeist.

3. Verfahren nach einem der Ansprüche 1 oder 2, dadurch gekennzeichnet, daß man ein Rücklaufverhältnis von (0,9 bis 1,2) : 1 einstellt.

4. Verfahren nach einem der Ansprüche 1 bis 3, dadurch gekennzeichnet, daß man die Veresterung in einer Destillationskolonne mit 30 bis 40 Siebböden vornimmt und die gesamte Essigsäure auf dem 20. bis 25. Siebboden zugibt, 60 bis 70 Gew% des insgesamt eingespeisten Methanols gasförmig in die Destillationsblase und das restliche Methanol 4 bis 6 Siebböden oberhalb der Essigsäureeinspeisung einführt.

5. Verfahren nach einem der Ansprüche 1 bis 4, dadurch gekennzeichnet, daß man als Veresterungskatalysator konzentrierte Schwefelsäure einsetzt.

6. Verfahren nach einem der Ansprüche 1 bis 5, dadurch gekennzeichnet, daß man die Destillation drucklos betreibt.

7. Verfahren nach einem der Ansprüche 1 bis 6, dadurch gekennzeichnet, daß man die Essigsäure zusammen mit dem Katalysator auf eine Temperatur von 40 bis 60°C erwärmt.

8. Verfahren nach einem der Ansprüche 1 bis 7, dadurch gekennzeichnet, daß man aus dem Wasser-Methanol-Essigsäure-Sumpf der Destillationsblase in einer zweiten Destillation das Methanol rückgewinnt.

9. Verfahren nach Anspruch 8, dadurch gekennzeichnet, daß man die zweite Destillation mit einem Rücklaufverhältnis von (5 bis 50) : 1, insbesondere (10 bis 15) : 1, betreibt.

10. Verfahren nach einem der Ansprüche 8 oder 9, dadurch gekennzeichnet, daß man das rückgewonnene Methanol in die Veresterungsstufe rückführt.

## Claims

1. A process for continuously producing an anhydrous methyl acetate/methanol mixture by esterifying acetic acid and methanol in the presence of an acidic esterification catalyst, which comprises carrying out the esterification in a distillation column having n = 20 to 50 distillation trays,
a) the acetic acid being fed together with the catalyst to a distillation tray which is at a distance of 0.5 n to 0.8 n from the still bottom,
b) feeding 50 to 80 % by weight of the total methanol to the still bottom and the remaining methanol to a distillation tray which is 2 to 10 trays above the acetic acid feed tray,
c) feeding methanol and acetic acid into the distillation column at a molar ratio of (1.5 to 3) : 1,
d) setting a reflux ratio of (0.75 to 1.5) : 1,
e) taking off the anhydrous methyl acetate/methanol mixture at the top of the column and
f) taking off a water/methanol/acetic acid bottom product with the catalyst from the still bottom.

2. The process as claimed in claim 1, wherein a methanol/ acetic acid mixture having a molar ratio of (1.6 to 2.0) : 1 is fed.

3. The process as claimed in either of claims 1 or 2, wherein a reflux ratio of (0.9 to 1.2) : 1 is set.

4. The process as claimed in any of claims 1 to 3, wherein the esterification is carried out in a distillation column having 30 to 40 sieve trays and all the acetic acid is added to the 20th to 25th sieve tray, 60 to 70 % by weight of the total methanol fed are introduced in the gaseous state into the still bottom and the remaining methanol is introduced 4 to 6 sieve trays above the acetic acid feed.

5. The process as claimed in any of claims 1 to 4, wherein concentrated sulfuric acid is used as the esterification catalyst.

6. The process as claimed in any fo claims 1 to 5, wherein the distillation is operated unpressurized.

7. The process as claimed in any of claims 1 to 6, wherein the acetic acid is heated together with the catalyst to a temperature of from 40 to 60°C.

8. The process as claimed in any of claims 1 to 7, wherein the methanol is recovered in a second distillation from the water/methanol/acetic acid bottom product from the still bottom.

9. The process as claimed in claim 8, wherein the second distillation is operated at a reflux ratio of (5 to 50) : 1, especially (10 to 15) : 1.

10. The process as claimed in claim 8 or 9, wherein the recovered methanol is recycled to the esterification stage.

## Revendications

1. Procédé de production continue d'un mélange anhydre d'acétate de méthyle et de méthanol par estérification d'acide acétique et de méthanol en présence d'un catalyseur d'estérification acide, caractérisé en ce qu'on procède à l'estérification dans une colonne de distillation comportant n = 20 à 50 plateaux de distillation et que dans ce cas
a) on introduit l'acide acétique avec le catalyseur sur un plateau de distillation dont l'écartement par rapport à la chaudière de distillation est de 0,5 n à 0,8 n,
b) on introduit 50 à 80 % en poids du méthanol total dans la chaudière de distillation et le reste du méthanol sur un plateau de distillation qui se trouve 2 à 10 plateaux au-dessus du plateau d'introduction de l'acide acétique,
c) on introduit le méthanol et l'acide acétique dans la colonne de distillation dans un rapport molaire de (1,5 à 3) : 1,
d) on ajuste le taux de reflux à (0,75 à 1,5) : 1,
e) on prélève le mélange anhydre d'acétate de méthyle et de méthanol en tête de la colonne et
f) on soutire de la chaudière de distillation un produit de queue composé d'eau, de méthanol et d'acide acétique ainsi que le catalyseur.

2. Procédé selon la revendication 1, caractérisé en ce qu'on introduit un mélange de méthanol et d'acide acétique ayant un rapport molaire de (1,6 à 2,0) : 1.

3. Procédé selon la revendication 1 ou 2, caractérisé en ce qu'on ajuste le taux de reflux à (0,9 à 1,2) : 1.

4. Procédé selon l'une des revendications 1 à 3, caractérisé en ce qu'on procède à l'estérification dans une colonne de distillation comportant 30 à 40 plateaux perforés et qu'on ajoute l'acide acétique total sur le plateau perforé n° 20 à 25 qu'on charge 60 à 70 % en poids du méthanol total introduit, sous une forme gazeuse, dans la chaudière de distillation et le reste du méthanol 4 à 6 plateaux perforés au-dessus de l'alimentation en acide acétique.

5. Procédé selon l'une des revendications 1 à 4, caractérisé en ce qu'on utilise de l'acide sulfurique concentré comme catalyseur d'estérification.

6. Procédé selon l'une des revendications 1 à 5, caractérisé en ce qu'on procède à la distillation en l'absence de pression.

7. Procédé selon l'une des revendications 1 à 6, caractérisé en ce qu'on chauffe l'acide acétique ainsi que le catalyseur à une température de 40 à 60°C.

8. Procédé selon l'une des revendications 1 à 7, caractérisé en ce qu'on récupère le méthanol dans le produit de queue de la chaudière de distillation, composé d'eau, de méthanol et d'acide acétique, au cours d'une seconde distillation.

9. Procédé selon la revendication 8, caractérisé en ce qu'on procède à la seconde distillation pour un taux de reflux de (5 à 50) : 1, notamment de (10 à 15) : 1.

10. Procédé selon la revendication 8 ou 9, caractérisé en ce qu'on recycle le méthanol récupéré à l'étape d'estérification.
